Europäisches Patentamt

European Patent Office

Office européen des brevets

(11) Publication number : **0 485 304 A2**

## (12) EUROPEAN PATENT APPLICATION

(21) Application number : **91403021.8**

(51) Int. Cl.⁵ : **C12P 19/14**

(22) Date of filing : **08.11.91**

(30) Priority : **08.11.90 JP 305463/90**

(43) Date of publication of application :
**13.05.92 Bulletin 92/20**

(84) Designated Contracting States :
**AT BE CH DE DK ES FR GB GR IT LI LU NL SE**

(71) Applicant : **MATSUTANI CHEMICAL INDUSTRIES CO. LTD.**
**3-Banchi, 5-chome, Kita Itami**
**Itami-shi, Hyogo-ken (JP)**

(72) Inventor : **Ohkuma, Kazuhiro**
**4-7, Yayoigaoka 3-chome**
**Sanda-shi, Hyogo-ken (JP)**
Inventor : **Matsuda, Isao**
**717-1, Noma Aza Raifukuzi**
**Itami-shi, Hyogo-ken (JP)**
Inventor : **Hanno, Yoshio**
**52-401, Ogino 3-chome**
**Itami-shi, Hyogo-ken (JP)**

(74) Representative : **Gillard, Marie-Louise et al**
**Cabinet Beau de Loménie 55, Rue d'Amsterdam**
**F-75008 Paris (FR)**

(54) **Method for preparing low caloric dextrin.**

(57) A method for preparing a low caloric dextrin of which caloric value is not more than 305Kcal/100g is provided using a pyrodextrin prepared by heating a starch to which mineral acid is added. The method comprising the steps of dissolving a pyrodextrin into water, and reacting alpha-amylase on the dextrin.

EP 0 485 304 A2

Jouve, 18, rue Saint-Denis, 75001 PARIS

## BACKGROUND OF THE INVENTION

### 1. Field of the Invention:

The present invention relates to a method for preparing a low caloric dextrin by treating pyrodextrin with an enzyme.

### 2. Description of Prior Art:

Recent years, living standard has been remarkably improved in Japan and, in particular, eating habits have come to substantially the same level as those in Western countries. As a result, average life of Japanese people has been prolonged, thereby bringing about rapidly an aging society which, in turn, results in structural change of disease sharply increasing diseases of adult people. Thus, promotion of health is now one of the matters of greatest concern. In this connection, the Health and Welfare Ministry of Japan published a proposal of "Guideline of Eating Habits for Promotion of Health" in 1985 with regard to eating habits of Japanese people in general, and in which it was pointed out that one of the problems pertaining to Japanese eating habits was "excessive intake of energy."

On the other hand, in various kinds of processed foods, starch itself and most of modified starches such as pregelatinized starch, pyrodextrin, starch derivative, glucose, corn syrup solid and maltodexitrin are actually employed in large quantity. Caloric value of these starch products, however, mounts to about 400Kcal/100g, and accordingly only pyrodexitrin is known among the starch products as a useful low caloric food material capable of saving the mentioned "excessive intake of energy."

The inventors of the present application have been intensely engaged in research and development of dietary fibers, and based on the results thereof already filed a patent application titled "method for preparing dextrin containing high percentage of dietary fibers" and others (JP.88.254 540). It has been heretofore known that pyrodextrin is a low caloric material, but is not adaptable to be employed as food material due to its stimulative taste, irritating smell, coloring difficulty, etc. The inventors, however, have come to conceive an idea of employing this pyrodextrin as a new food material. To realize this new idea, the inventors have been further intensely engaged in studies of overcoming the mentioned drawbacks of pyrodextrin aiming at a satisfying low caloric food material therefrom while maintaining the property of low calorie as it is. The inventors further have attempted to develop means for easy mass production.

## SUMMARY OF THE INVENTION

Accordingly, it is an object of the present invention to provide a novel method for preparing a low caloric dextrin.

To accomplish the foregoing object, the method for preparing a low caloric dextrin in accordance with the invention includes the processes of determining conditions to be satisfied by pyrodextrin used as a raw material of the invention; and extracting the fraction of digestion by either chromatographic method or organic solvent method if lower calorie is required, thereby obtaining a low caloric dextrin.

The dextrin in accordance with the invention is produced in the following methods:

(1) A method comprising the steps of: heating a potato starch by treatment with mineral acid thereby obtaining a pyrodextrin; reacting alpha-amylase with the pyrodextrin obtained; and refining the pyrodextrin thus treated with alpha-amylase by filtering according to normal method.

(2) A method comprising the steps of: reacting alpha-amylase with an ordinary pyrodextrin; applying glucoamylase to the hydrolyzate; refining the resulted hydrolyzate by filtering; and extracting the digestive section.

Described first is the above method (1).

It is required to use a quite limited kind of pyrodextrin as a raw material. That is, only a potato starch is qualified as starch material. It is essential to add a mineral acid as a catalyst in the heating step. Various mineral acids are known, and from the viewpoint of use in food, it is particularly desirable to adopt hydrochloric acid. In the pyrodextrin thus obtained, caloric value should be not more than 305Kcal/100g in view of application to prepare a low caloric food. In other words, pyrodexrin whose content corresponds to not more than 70% of 400Kcal/100g of ordinary starch or starch product should be used as the raw material. In this respect, it is to be noted that caloric value of white dextrin popularly used in food and medical field exceeds far from the mentioned 305Kcal/100g, and is not suitable for the purpose of achieving low calorie. On the other hand, if the caloric value is less than 257kcal/100g, the food even though hydrolized with alpha-amylase exhibits somewhat a stimulative taste. Moreover, the load in the refining step after hydrolysis with alpha-amylase is enhanced thereby making it impossible to carry out mass production. Thus, such dextrin of excessively low caloric value is not suitable, either.

Various alpha-amylases can be widely employed in the present invention including bacterial alpha-amylase. From the viewpoint of reducing calorie of dextrin in the object, it is particularly preferable to use "Thermamyl" (produced by Novo Ind.) and "Kleistase T-5" (produced by Daiwa Kasei Co.).

The low caloric dextrin thus obtained has usually a caloric value within a range of not more than 305Kcal/100g and not less than 257Kcal/100g which is quite preferable as a low caloric food material.

Potato starch treated with mineral acid and heated according to normal method is preferably employed as a pyrodextrin treated with mineral acid to be used in the mentioned method (1). As for the amount of mineral acid, 3 to 10 % of about 1% aqueous solution is added to the starch. To satisfy the heating requirement, considering that an acidified aqueous solution was already added, the starch and acid are evenly mixed by stirring and aging in a mixer or disintegrator, then the mixture is preliminarily dried at 100 to 120°C to reduce moisture content to about 5%, and heated at a temperature of 150 to 200°C for 1 to 4 hours. Treatment of the pyrodextrin with alpha-amylase can be carried out according to conventional method, and in which about 30 to 45% aqueous solution of pyrodextrin is prepared with its pH adjusted to 4.5 to 6.5, then alpha-amylase is added to the pyrodextrin by 0.05 to 0.2%, and the pyrodextrin is held for 30 min to 2 hours at 85 to 100°C, i.e., a reaction temperature of alpha-amylase (this temperature depends on the type of alpha-amylase). In the next step, reaction of alpha-amylase is suspended by increasing the temperature to about 120°C (i.e., deactivation temperature of alpha-amylase). After completing the reaction of alpha-amylase, activated charcoal is added for the purpose of removing insoluble matter, color, etc. Then filtration is carried out by means of conventional filter press, precoat filter, or the like. Thereafter, salts and coloring matter in the solution are removed by ion exchanger resins. Usually, a cation exchange resin, an anion exchange resin and a mixed bed of both types are applied in this order.

Described now is the mentioned method (2).

In this method, not only pyrodextrin prepared from potato starch but also other pyrodextrins can be widely employed. Any other pyrodextrin prepared from various starches as corn starch, tapioca starch and the likes can be employed. Treatment with alpha-amylase is carried out in the same manner as the foregoing method (1), but in this method (2), after completing such treatment, a further treatment with glucoamylase is required, and in which conventional conditions for such treatment with glucoamylase is adopted. For example, a solution temperature is reduced to about 55°C with its pH adjusted to about 5.5, then 0.05 to 0.2% by weight of commercial glucoamylase is added to the original pyrodextrin, and reaction takes place for 24 to 48 hours keeping the solution at this temperature. This reaction is used to decompose small molecules such as oligosaccharide existing in the solution to glucose. Then the reaction of glucoamylase is completed at 80°C, for example.

Subsequently, filtration and refining are carried out according to conventional method.

High caloric fraction is then separated and removed using ion exchange resin by chromatographic method and/or organic solvent method. For that purpose, any commercial strongly acidic cation exchange resin can be widely used.

Preferable examples are Amberlite IR-116, IR-118, IR-120B, XT-1022E, XT-471F (all manufactured by Organo), Diaion SK-1B, SK-102, SK-104, SK-106, SK-110, SK-112, SK-116, FR-01 (all manufactured by Mitsubishi Chemicals), and XFS-43281.00, XF-43280.00, XFS-43279.00, XFS-43278.00 (all manufactured by Dow Chemical ).

These resins are preferably dealt with as alkaline metal type or alkaline earth metal type before their uses. It is preferable to adjust the rate of flow of the column fluid according to the resin used. The rate of flow of the fluid is preferably in the range of S.V. = 0.1 to 0.6. A rate of flow out of the above range tends to deteriorate the workability and separability. The temperature at the time of running of the fluid is preferably in the range from 20°C to 70°C, and a temperature below this range will deteriorate the separability and make the viscosity of fluid increase, thereby yielding negative influence on the fluid, while a temperature exceeding this range will cause the fluid to be colored and deteriorate other quality characteristics.

The mentioned organic solvent method is a separation method using solvent capable of dissolving digestible or low molecular weight components, and accordingly is preferable to adopt a solvent capable of dissolving low molecular weight components. Preferable as a representative example of such solvent is ethanol.

In the mentioned method (2), since reaction with glucoamylase takes place after hydrolysis with alpha-amylase, not only the pyrodextrin prepared from potato starch is applicable but also various other pyrodextrins can be equivalently used to obtain a dextrin of desired low caloric value, i.e., not more than 305Kcal/100g, more preferably, not more than 257Kcal/100g. Since glucoamylase is additionally applied after hydrolysis with alpha-amylase in the method (2), stimulative substances in the object dextrin of not more than 257Kcal/100g can be also successfully separated by normal method in the steps of filtration and refining.

Several experimental examples are hereinafer described in order to show clearly the features of the present invention.

Experimental Example 1

50ml of 1% hydrochloric acid solution per 1 kg of various commercial starches were applied by spraying on said starches, mixed evenly by means of a mixer, placed on an aluminum vat, pre-dried for 1 hour in a drier, then heated at a temperature of 150°C for 2 hours. Warm water at a 2:1 ratio to pyrodextrin was added to each pyrodextrin and neutralized to pH5.8 with 1N sodium hydroxide; then 0.1% "Thermamyl" was added to each solution to react at a temperature of 95°C for 1 hour, and further heated to 115°C to complete the reaction. Subsequently, each solution was filtered and decolorized and concentrated in vacuo to a concentration of 30%. Thereafter, caloric value of each solution together with intermediate products was determined, and transparency of each sample solution was measured. Table 1 shows the result.

TABLE 1

| Material starches | Potato starch | Tapioca | Corn starch |
|---|---|---|---|
| Kcal/100g of pydrodextrin | 262 | 283 | 283 |
| Kcal/100g after hydrolysis with alpha-amylase | 269 | 288 | 281 |
| Transparency of solution after refining | transparent | transparent but reddish | opaque |

Experimental Example 2

50ml of 1% hydrochloric acid solution per 1 kg of commercial potatoe starches were applied by spraying on said starches and mixed evenly by means of a mixer, placed on an aluminum vat, pre-dried for 1 hour in a drier, then heated at a temperature of 150°C for 5 hours while picking up 800g of sample every hour . Warm water at a 2:1 ratio to pyrodextrin was added to each pyrodextrin and neutralized to pH5.8 with 1N sodium hydroxide; then 0.1% "Thermamyl" was added to each solution to react at a temperature of 95°C for 1 hour, and further heated to 115°C to complete the reaction. Subsequently, the solution decolorized and filtered by conventional method was subject to a deionization test using mixed bed ion exhange resins. Indication of successful deionization occurs when chloride leaks into the effluent. Caloric value of the deionized solution was determined after being concentrated in vacuo to 30%, and flavor of each solution was measured by sensory test. Table 2 shows the results.

4

TABLE 2

| Heating time | 1 | 2 | 3 | 4 | 5 |
|---|---|---|---|---|---|
| Amount of deionized effluent by ion exchange resin (ml/ml resin) | 7.31 | 5.54 | 4.30 | 3.71 | 2.69 |
| Kcal/100g | 343 | 310 | 267 | 260 | 255 |
| Flavor (stimulative taste) | good | good | good | good | excessively stimulative |

Experimental Example 3

0.1% "Kleistase KD" was added to each 400g of pyrodextrin obtained in the foregoing Example 2 and reacted at 85°C. Other treatments and conditions were the same as those described above. Table 3 shows the result of determination of caloric values.

TABLE 3

| Heating time | Kcal/100g |
|---|---|
| 1 | 341 |
| 2 | 321 |
| 3 | 281 |
| 4 | 276 |
| 5 | 275 |

It is understood from Table 1 that caloric value of 3 kinds of starches are less than 288 Kcal/100g; that a transparent solution can be obtained when using potato starch; that use of tapioca starch results in a reddish solution; and that solution using corn starch is opaque. This means that the latter two starches are not suitable for food.

However, when extracting digestive fractions using ion exchange resin chromatography or organic solvent method by reacting glucoamylase with an aqueous solution of pyrodextrin after reacting alpha-amylase therewith, then filtering and refining it by conventional method, the colored and/or opaque substances can be removed, and therefore starch other than potato starch such as corn starch can be also employed as a raw material.

It is also understood from Table 2 that, though caloric value reduces in proportion to the length of heating time, capacity of ion exchange resin reduces here caloric value down to less than 257Kcal/100g. Since the capacity is one of the important steps in refining process, such reduction of capacity is not desirable for mass production, and moreover there arises a further disadvantage of stimulative taste impossible to remove even by ion exchange resin.

Comparing the caloric values between Table 2 and 3, it is understood that lower calories are achieved in the dextrin prepared by adding "Thermamyl", and therefore it is obviously preferable to use "Thermamyl", as far as caloric value is in the mentioned range of 257 to 305Kcal/100g. In addition, measurement of the caloric value was carried out in the same manner as a later described embodiment.

Note that the low caloric dextrin achieved in accordance with the invention can be desirably applied to a variety of foods including, but not limited to, breads and confectioneries such as cookies, doughnuts, cakes, breads; creams such as custard cream, cream, butter cream; and a variety of other foods such as chocolate, chewing gum, pudding, Bavarian, jelly, yoghurt, ice cream, juce, milk shake.

DESCRIPTION OF THE PREFERRED EMBODIMENTS

Several embodiments in accordance with the present invention are hereinafter described. In these embodiments, measurement of caloric values were carried out by the following method.

1. Method for measuring caloric value

Measurements were carried out according to the following method titled "Measurement of Physiological Combustion Heat of Food for Specified Health Use Containing Water-Soluble Low Caloric Sugars" which is notified by the Ministry of Health and Welfare of Japan.

1-1. Reagent and others

(1) Somogy copper Reagent:

90g of potassium sodium tartrate and 225g of $Na_3PO_4$ $12H_2O$ were dissolved into water, then 30g of $CuSO_4 \cdot 5H_2O$ and 3.5g of KI were sequentially added, then filled up with distilled water to 1000 ml.

(2) Lowry Reagent:

Solution A: prepared by mixing 1% $CuSO_4 \cdot 5H_2O$ and 2.2% Potassium sodium tartrate by the ratio of 1 to 1.

Solution B: prepared by mixing phenol and distilled water by the ratio of 1 to 0.8.

(3) Sugar-alcohol measuring kit:

F-kit: F-kit for D-sorbitol/xylitol (produced by Boeringer Manheim Yamanouchi)

(4) Diastase solution:

2% diastase (Japanese Pharmacopoeia) solution.

(5) Hydroxyl amine pyridine solution: prepared by dissolving 100ml of hydroxyl amine into 10ml of pyridine.

(6) Conditions of gas chromatography

FID gas chromatograph: 5% SE30 chromosorb W: glass column or stainless steel column of 3 to 4mm of internal diameter and 2m in length: column temperature 185°C: and carrier gasflow of 80 ml/min.

1-2. Measurement of Total Water-Soluble Reducing Sugars

(1) Preparation of a test solution:

If a sample includes oligosaccharides alone, they are extracted using water or 80% ethanol, and if including polysaccharides, then oligosaccharides should be completely extracted using 80% ethanol. The extracted solution was then concentrated under reduced pressure (at a temperature lower than 60°), and the residue was completely dissolved into a small amount of 50mM maleic acid-Na buffer to finally obtain about 500mg of glucose.

(2) Procedure:

1N-hydrochloric acid was added to the test solution by the ratio of 2:1 and heated at 100°C for 20 hours in a boiling water bath. After cooling, the solution was neutralized with 1N-sodium hydroxide (bromothymol blue test paper). Then the reducing sugars of the obtained solution were measured by Somogy method, and 5,6 carbonic sugar alcohol was measured by gas chromatography or F-kit. The sun of the sugars thus obtained is the total amount of water-soluble sugars (A).

(3) Somogy method:

2.5ml of Somogy solution was added to 7.5ml of the test solution (which comprises 1 to 10mg of reducing sugar) and heated at 100°C for 10 minutes. After cooling, 2ml of 5% KI and 3ml of 1.2N-sulfuric acid were added to the solution and mixed well. Then this mixture was titrated with $1/40N\text{-}Na_2SO_3 \cdot 5H_2O$. Glucose was used as a standard sugar. The amount of sugars was obtained by a value thus titrated.

(4) Measurement of sugar-alcohol:

The amounts of sorbitol and xylitol were determined with the use of F-kit by appropriately diluting the test solution which had been hydyolyzed for the purpose of the mentioned determination of total amount of water-soluble sugars.

In case of containing pentose and hexose alcohol other than sorbitol and xylitol, the determination was carried out by gas chromatography.That is, the test solution which had been hydrolyzed for the determination of the total amount of water-soluble sugars, was concentrated under reduced pressure at a temperature lower than 60°C and, after adding ethanol so as to obtain a final concentration exceeding 80%, the solution was heated and extracted in a boiling water bath for 30 minutes. The extract was concentrated in vacuo at a temperature lower than 60°. 80% ethanol was added to this concentrated extract to obtain a specified amount thereof. Picking up 5ml of thesolution thus obtained, the solvent was completely removed therefrom under reduced pressure. The residue was dissolved into 1 ml pyridine, then 1 ml of hydroxyl amine pyridine was further added, and, after leaving this solution for 5 minutes as it was, the solvent was removed under reduced pressure. 1 ml of benzene was added to the residue, and after removing completely $H_2O$ under reduced pressure, the residue was dissolved by adding 2 ml of pyridine. After the dissolution, 0.2 ml of hexamethyl disilane and 0.1 ml of trimethyl silane were added to the solution. After shaking for well mixing, the solution was left for 15 minutes at room temperature, and a specified amount of this solution was obtained by removing pyridine and was then determined by absolute calibration curve method using gas chromatography.

1-3 Determination of insoluble starch

(1) With respect to a sample containing starch, using a sample corresponding to 2.5 to 3.5 mg of dry material, 80% ethanol extracted residue obtained in the same manner as the mentioned extraction method of total water-soluble sugars was dispersed into 200ml water, which was then soaked into a boiling water bath and heated for 15 minutes while being continuously stirred. Then, the solution was cooled down to 55°C and, after adding 10ml of diastase solution, left for one hour at 55°C. Subsequently, after boiling for several minutes, the solution was again cooled down to 55°C, then, adding 10 ml of diastase solution, was stirred well and left for one hour. At this time, when the residue in the reaction solution showed a positive reaction to the iodine starch reaction, diastase solution was further added, then digestion takes plase. Distilled water was added to the diastase treated solution of which iodine starch reaction was negative, thereby obtaining 250ml of mixed solution with distilled water, and the mixture was then filtered through a filter paper. Hydrochloric acid was added to the filtered solution so that the mixture was a 2.5% solution, and heated in a boiling water bath for 2.5 hours. After cooling, the solution was neutralized with 10 % sodium hydroxide, then the filtered solution was adequately diluted, and glucose was determined by Somogy method. The amount of glucose thus obtained was multiplied by 0.9 to obtain a starch amount (A').

1-4. Determination of sugars digested and absorbed in the small intestine

(1) Determination of sugars digested by an enzyme.

1) Preparation of a solution of a commercially available rat small intestinal acetone powder

0.9% NaCl was added to a rat small intestinal acetone powder produced by Sigma to obtain a suspension (100mg/ml). After conducting an ultrasonic treatment (60 sec, 3 times), the solution was centrifuged (3000 rpm, 30 min) and a supernatant thereof was an enzyme solution. The protein amount of this enzyme solution was determined by Lowry method. The solution was then subject to an adjustment so that the enzyme activity thereof was about 0.1mg/mg protein/hour as activity of sucrose hydrolysis.

2) Digestion test using a rat small intestinal acetone powder

Extract of 80% ethanol prepared for determination of the mentioned total amount of water-soluble sugars, or extract of water was concentrated, then diluted by 50mM maleic acid-Na buffer (pH 6.0) so that the concentration of sugars be 1 to 4%, to obtain thus a sample. 1.0 ml of this solution and 1.0 ml of the enzyme solution were mixed and incubated for one hour at 37°C. After the incubation, the solution was heated for deactivation in a boiling water bath for 10 minutes. And after the centrifugation (3000 rpm, 30 min), with respect to the supernatant, the amount of the reducing sugars was determined by Somogy method while the amount of sugar alcohol was measured by F-kit or gas-chromatography, the sum of these two measured amounts being an amount of enzymatic digestive absorption sugars. At this time, the reducing sugars and sugar alcohol which existed as monosaccharide in the sample from the beginning were also measured. The decomposition percentage was obtained by dividing the amount of digestive absorption sugars by the total amount of water-soluble sugars. As a control experiment, the same process as the foregoing was conducted with respect to sucrose or maltose of the same amount of water-soluble sugars. In this control experiment, the decomposition percentage had to be more than 20% when the experiment was conducted on sucrose. Ratio of the mentioned decomposition percentage on sucrose or maltose to a decomposition percentage obtained from the tested sugars was that of a small intestine digestive absorption percentage. This small intestine digestive absorption percentage was then multiplied by the total amount of water-soluble sugars to obtain a total amount of digestive absorption reducing sugars (B), and also multiplied by the total amount of sugar alcohol to obtain a total amount of digestive absorption sugar alcohols (C).

A control sugar selecting whether sucrose or maltose is to be employed is performed by the following method: That is, a digestion test was carried out employing maltose as a substrate in half amount of the tested sugars under the same conditions as the mentioned digestion test by a rat small intestinal acetone powder. In case glucose created by the digestion was not exceeding 10 times as much as the sum of sugars and sugar alcohol created in the digestion test of the tested sugars, maltose was employed as a control sugar. When exceeding 10 times, sucrose was employed.

3) Determination of protein

0.3 ml of 1N-sodium hydroxide was added to 0.1 ml of sample (containing 20 to 100 μg of protein), and left for 15 minutes. 3.0 ml of the mentioned solution A was further added and left for 10 minutes at room temperature. Then, 0.30 ml of the mentioned solution B was added, and after 30 minutes, the light absorbancy at a wave-length of 750nm was measured. Cattle serum-albumin was employed as standard protein.

5. Calculation of physiological Combustion Heat Value

The physiological combustion heat value can be said a sum of the effective energies generated by the digestive absorption and the enzymatic absorption. Accordingly, the physiological combustion heat value can be calculated as follows:

The physiological combustion heat value (kcal)/g) = (Starch A') x 4 + (Total amount of digestive absorption reducing sugars (B)) x 4 + (Total amount of digestive absorption sugar alcohol (C)) x 2.8 + (Total water-soluble sugars A) - (Total amount of digestive absorption reducing sugars (B) + Total amount of digestive absorption sugar alcohols (C) x 0.5 x 1.9

Example 1

2500kg of potato starch was put into a Ribbon Mixer, 250 liters of 1% hydrochloric acid were sprayed with compressed air while rotating the mixer, and after being uniformized through a disintegrator, further allowed to age in the Ribbon-mixer for 10 hours. The obtained mixture was pre-dried to 3% moisture, subsequently put into a Rotary-Kiln-Type converter to be continuously heated at a temperture of 180°C for two hours. It was acknowledged that the pyrodextrin thus obtained had a caloric value of 264kcal/100g.

4000 liters of water were added to this pyrodextrin and its pH adjusted to 6.0 by adding 20% sodium hydroxide, then 0.2% by weight dry solids of the solution of alpha-amylase (Thermamyl 60L produced by Novo Inc.) added to hydrolyze at a temperature of 95°C for 1 hour. Most of the solution was then refined through conventional process such as decoloring and filtration with activated charcoal, deionization with ion exchange resins, and subsequently spray dried. Thus, about 1800kg of dextrin having a caloric value of 262kcal/100g was obtained.

Example 2

About 100 liters of residual solution hydrolyzed with alpha-amylase in the foregoing Example 1 were heated at a temperature of 55°C, with its pH adjusted to 5.5, and saccharified by adding 0.1% by weight of glucoamylase (produced by Daiwa Kasei Co.). The pH was then adjusted to 3.5 and reaction of glucoamylase was terminated. After refining in the same manner as Example 1, 60kg of 50% solution was obtained through concentration. 100 liters of this solution was applied, at S.V.=0.25, to a column packed with "XFS-43279.00" (produced by Dow Chemical Japan), an alkali metal type strongly acidic cation exchange resin, then the high molecular weight dextrin was extracted by applying water, and, after concentrating, a dextrin having a caloric value of 184kcal/100g was obtained by spray drying.

Example 3

25 liters of 95% ethanol were added to 1 liter of 50% concentrated solution obtained in Example 2 while being stirred, then left for 1 hour, centrifugated to separate the precipitates, dried in vacuo at 70°C, with dried dextrin solid obtained.

Example 4

2500kg of commericial corn starch was put into a ribbon mixer. 250 liters of 1% hydrochloric acid solution was sprayed using compressed air while rotating the mixer. After uniformized by a disintegrator, the mixture was aged for 10 hours. After pre-drying the mixture to be about 4% moisture by a flash drier, the mixture was consecutively put in Rotary-Kiln-Type converter and heated at 180°C for 2 hours. Caloric value of the pyrodextrin thus obtained was 238kcal/100g per solid. Then, a solution of this pyrodextrin was heated to 55°C, with its pH adjusted to 5.5, and was saccharified by adding 0.1% by weight of glucoamylase (produced by Daiwa Kasei Co.). The pH was then adjusted to 3.5 and reaction of glucoamylase was terminated. Then, after refining through conventional process of decoloring and filtration with activated charcoal and deionization with ion exchange resins, the solution was concentrated to 50% and applied, at S.V.=0.25, to a column packed with 2500 liters of "XFS-43279.00" (produced by Dow Chemical Japan), which was an alkali metal type strongly acidic cation exchange resin, the high molecular weight dextrin was extracted by applying water, and after concentrating, a dextrin having a caloric value of 184kcal/100g was obtained by spray drying.

**Claims**

(1) A method for preparing a low caloric dextrin of which caloric value is 257 to 305Kcal/100g comprising the steps of: dissolving a pyrodextrin into water, said pyrodextrin being prepared by heating a potato starch to which mineral acid is added; and reacting alpha-amylase on said pyrodextrin.

(2) A method for preparing a low caloric dextrin of which caloric value is not more than 257Kcal/100g comprising the steps of: reacting alpha-amylase with an aqueous solution of pyrodextrin; performing filtration and refining the mixed solution by conventional process; and extracting a digestive fraction by either ion exchange chromatography or organic solvent method.